# EUROPÄISCHE PATENTSCHRIFT

(11) **EP 2 764 877 B1**
(45) Veröffentlichungstag und Bekanntmachung des Hinweises auf die Patenterteilung: **06.04.2016**
(21) Anmeldenummer: 14153205.1
(22) Anmeldetag: 30.01.2014
(51) Int. Cl.: A61L 31/02, A61L 31/08, A61F 2/86, A61F 2/90, A61F 2/88

(54) **Intravaskuläres Funktionselement und System mit einem solchen Funktionselement**
Intravascular functional element and system with such a functional element
Élément fonctionnel intravasculaire et système doté d'un tel élément fonctionnel

(30) Priorität: 11.02.2013 DE 102013101337
(43) Veröffentlichungstag der Anmeldung: 13.08.2014
(73) Patentinhaber: Acandis GmbH & Co. KG, 76327 Pfinztal (DE)
(72) Erfinder: Cattaneo, Giorgio, 76199 Karlsruhe (DE); Mailänder, Werner, 75331 Engelsbrand Grunbach (DE)
(74) Vertreter: Kilchert, Jochen

(56) Entgegenhaltungen:
- US-A1- 2003 153 971
- US-A1- 2011 009 954
- Y. CHENG ET AL: "Surface characterization and mechanical property of TiN/Ti-coated NiTi alloy by PIIID", SURFACE AND COATINGS TECHNOLOGY, Bd. 201, Nr. 15, 27. August 2006 (2006-08-27), Seiten 6869-6873, XP055124943, ISSN: 0257-8972, DOI: 10.1016/j.surfcoat.2006.09.055
- NEELAKANTAN L ET AL: "Selective surface oxidation and nitridation of NiTi shape memory alloys by reduction annealing", CORROSION SCIENCE, OXFORD, GB, Bd. 51, Nr. 3, 17. Dezember 2008 (2008-12-17), Seiten 635-641, XP025962848, ISSN: 0010-938X, DOI: 10.1016/J.CORSCI.2008.12.018 [gefunden am 2009-02-23]

## Beschreibung

Die Erfindung betrifft ein intravaskuläres Funktionselement, insbesondere Stent und ein System mit einem solchen Funktionselement.

In der Medizintechnik werden Stents in der Regel durch Laserverfahren hergestellt. Es kommen aber auch Geflechte aus Nitinol-Drähten für Implantate (z.B. Stents oder Okkluder) zum Einsatz. Anders als bei durch Laserverfahren hergestellten Stents, gleiten bei Drahtgeflechten die Drähte übereinander und ermöglichen daher eine gute Verformung der Stent-Strukturen. Grundsätzlich können (vaskuläre) Implantate aus Halbzeugen, wie Blechen, Präzisionsrohren oder Drähten gefertigt werden.

Beispielsweise beschreibt US 2004/0117001 A1 ein Verfahren zur Herstellung eines Stents aus Nitinol. Ein Ziel der US 2004/0117001 A1 besteht darin, den Nickel-Gehalt in einer oberflächennahen Schicht zu reduzieren, um zu verhindern, dass Nickel aus der Schicht freigesetzt wird, da dadurch die Biokompatibilität des Stents beeinträchtigt wird. Zur Herstellung der Stents wird ein Laser-Verfahren vorgeschlagen. Nach einem Kaltbearbeitungsschritt wird der Stent wärmebehandelt und dann bei Temperaturen unter 20°C elektropoliert. Zur thermischen Oxidierung wird der Stent mit Heißdampf bei einer Temperatur von 150°C für 12h beaufschlagt. Dadurch soll eine oxidische Oberfläche mit einem Ni-Gehalt von weniger als 2 Gew.% in einer Schichttiefe von 10 nm erreichbar sein.

US2003/0153971 A1 beschreibt ein Intravaskuläres Implantat wie z.B. einen Stent, mit einer Gitterstruktur, die mehrere Drähte jeweils aus einer Legierung mit den Legierungselementen Nickel und Titan aufweist, wobei die Gitterstruktur in Längsrichtung und in Umfangsrichtung jeweils Zellen aus sich kreuzenden Drähten bildet und in Umfangsrichtung mindestens 6 Zellen sind. Jeder der Winkel zwischen den Drähten und einer in Längsrichtung der Gitterstruktur verlaufenden Längsachse beträgt mindestens 45°. Y. Cheng und Y. F. Zheng beschreiben in "Surface characterization and mechanical property of TiN/Ti-coated NiTi alloy by PIIID" (SURFACE AND COATINGS TECHNOLOGY; Bd. 201, Nr. 15, 27. August 2006 (2006-08-27), Seiten 6869-6873), die Oberflächemodifikation einer NiTi Legierung durch PIIID Technik. Die auf der Substratoberfläche gebildete Oxidschichtist nickelarm und enthält Ti02, Titanitrid und Titanoxinitrid. NEELAKANTAN L. et al beschreiben in "Selective surface oxidation and nitridation of NiTi shape memory alloys by reduction annealing" (CORROSION SCIENCE, OXFORD, GB; Bd. 51, Nr. 3, 1. März 2009 (2009-03-01), Seiten 635-641) die Herstellung von einer Mischoxidschicht auf der Oberfläche einer NiTi-Legierung, wobei die Mischoxidschicht TiO2 und Titanitrid enthält.

Das bekannte Verfahren hat den Nachteil, dass die damit herstellbaren Oxidschicht bei Implantaten, die aus Drahtgeflechten bestehen, schnell verschleißen. Bei Geflechten kommt es darauf an, dass die Kontaktflächen der sich berührenden Drähte reibungsarm sind, gerade wenn man bedenkt, dass die implantierten Geflechte permanent pulsatilen Gefäßbewegungen ausgesetzt sind und sich die Drähte deshalb relativ zueinander bewegen. Ein niedriger Reibungskoeffizient der Drahtoberflächen ist überdies für die gute Bewegbarkeit des Implantats in einem Zufuhrkatheter wichtig.

Es ist Aufgabe der Erfindung, ein intravaskuläres Funktionselement, insbesondere Stent, mit verbessertem Verschleiß- und Reibungsverhalten und guter Biokompatibilität anzugeben. Eine weitere Aufgabe der Erfindung ist es, ein System mit einem solchen Funktionselement anzugeben.

Diese Aufgabe wird im Hinblick auf das Funktionselement durch den Gegenstand des Anspruches 1 und im Hinblick auf das System durch den Gegenstand des Anspruch 13 gelöst.

Insbesondere wird die Aufgabe durch ein intravaskuläres Funktionselement mit einer röhrchenförmigen Gitterstruktur gelöst, die einen Draht oder mehrere Drähte jeweils aus einer Legierung mit den Legierungselementen Nickel und Titan aufweist. Eine Mischoxidschicht ist auf der Oberfläche des Drahtes bzw. auf der Oberfläche der Drähte mit einer Schichtdicke von 15nm bis 100nm ausgebildet, die TiO2 und wenigstens ein Nitrid, insbesondere Titanoxinitrid und/oder Titanitrid aufweist. Die Gitterstruktur bildet in Längsrichtung und in Umfangsrichtung jeweils Zellen aus sich kreuzenden Drähten oder Drahtsegementen des Drahtes. In Umfangsrichtung der Gitterstruktur sind mindestens 6 Zellen, insbesondere mindestens 12 Zellen, insbesondere mindestens 16 Zellen, insbesondere mindestens 24 Zellen, insbesondere mindestens 36 Zellen, insbesondere mindestens 48 Zellen angeordnet. Für jede der vorstehend genannten Untergrenzen beträgt der Winkel α zwischen den Drahtsegmenten oder Drähten und einer in Längsrichtung der Gitterstruktur verlaufenden Längsachse zumindest abschnittsweise jeweils mindestens 45°, insbesondere mindestens 50°, insbesondere mindestens 55°, insbesondere mindestens 60°, insbesondere mindestens 65°, insbesondere mindestens 70°, insbesondere mindestens 75°.

Die Gitterstruktur kann ein Geflecht sein, bei dem die Drahtsegmente des Drahtes oder die Drähte miteinander verflochten sind. Bei dem Geflecht entspricht der Winkel α einem Flechtwinkel α.

Die Obergrenze der Zellenanzahl auf dem Umfang kann 48 Zellen betragen. Die Obergrenze des Winkels α kann 75° betragen.

Die Anzahl der Zellen auf dem Umfang wird durch die Anzahl der Drähte oder die Anzahl der Drahtsegmente bestimmt, die die Gitterstruktur bilden. Die Anzahl der Drähte oder die Anzahl der Drahtsegmente wird dadurch ermittelt, dass eine imaginäre Schnittfläche die Gitterstruktur senkrecht zu deren Längsachse schneidet. Die Schnittfläche verläuft also in radialer Richtung bezogen auf die röhrchenförmige Gitterstruktur. Die Anzahl der Drahtschnittpunkte in der Schnittfläche entspricht der Anzahl der Drähte, insbesondere Einzeldrähte, bzw. der Anzahl der Drahtsegmente der Gitterstruktur. Allgemein sind doppelt so viele Drähte oder Drahtsegmente wie Zellen vorhanden, wobei eine erste Hälfte der Drähte oder Drahtsegmente in einer ersten Spiralrichtung und eine zweite Hälfte der Drähte oder Drahtsegmente in einer zweiten Spiralrichtung entgegengesetzt zur ersten Spiralrichtung verlaufen, so dass sich die Drähte oder Drahtsegmente kreuzen und Zellen bilden.

Bspw. werden 6 Zellen auf dem Umfang durch 12 Drähte oder Drahtsegmente gebildet, wobei 6 Drähte oder Drahtsegmente im Uhrzeigersinn und 6 Drähte oder Drahtsegmente im Gegenuhrzeigersinn umlaufen.

Die Gitterstruktur kann aus mehreren Einzeldrähten gebildet sein, die ohne Umlenkung an den beiden axialen Enden der Gitterstruktur, d.h. jeweils mit freien, bzw. offenen Drahtenden verlaufen und zur Bildung der Zellen sich kreuzen, insbesondere verflochten sind. Es ist auch möglich, dass die Gitterstruktur aus einem einzigen Einzeldraht gebildet ist, der an beiden axialen Enden der Gitterstruktur umgelenkt ist und dort jeweils Schlaufen bildet. Die Zellen sind durch Drahtsegmente oder Drahtabschnitte des Einzeldrahtes gebildet, die in unterschiedlichen Spiralrichtungen auf dem Umfang des Funktionselements verlaufen und zur Bildung der Zellen sich kreuzen, insbesondere verflochten sind. Ferner kann die Gitterstruktur aus mehreren Einzeldrähten gebildet sein, die zumindest an einem axialen Ende der Gitterstruktur umgelenkt sind und sich kreuzende, insbesondere verflochtene Drahtsegmente bilden. Damit kann dieselbe Zellenanzahl auf dem Umfang bei verschiedenen Funktionselementen durch unterschiedlich viele Einzeldrähte gebildet sein, je nachdem, wie viele Einzeldrähte an einem oder an beiden axialen Enden der Gitterstruktur umgelenkt sind. Wenn die Gitterstruktur durch einen einzigen, mehrfach umgelenkten Einzeldraht gebildet ist, sollte die Anzahl der Zellen auf dem Umfang auf maximal 16 Zellen beschränkt sein, da die Herstellung der Gitterstruktur sonst sehr zeitaufwändig ist.

Erfindungsgemäß beträgt der Winkel α, insbesondere der Flechtwinkel α, zumindest abschnittsweise ≥ 45°, vorzugsweise ≥ 50°, vorzugsweise ≥ 55°, vorzugsweise ≥ 60°, vorzugsweise ≥ 65°, vorzugsweise ≥ 70°, vorzugsweise ≥ 75°, insbesondere maximal 75°. Mit steigendem Flechtwinkel nimmt die Flexibilität des Geflechts zu.

Der Flechtwinkel bezieht sich in diesem Zusammenhang auf den Ruhezustand der des Funktionselements (im expandierten Zustand). Die Bestimmung des Flechtwinkels wird also im entspannten Zustand ohne Einwirkung äußerer Kräfte vorgenommen. Dieser Zustand kann beispielsweise dem Herstellzustand entsprechen. Zur Bestimmung des Flechtwinkels ist das Funktionselement in axialer Richtung geradlinig ausgerichtet. In diesem Zusammenhang wird als Flechtwinkel derjenige Winkel bezeichnet, der zwischen einem Draht und einer senkrechten Projektion der Rotationsachse auf die Umfangsebene der Gitterstruktur bzw. des Geflechts gebildet ist. Die Rotationsachse entspricht der Längsachse des Geflechts bzw. der Gitterstruktur.

Konkret wird offenbart, dass bei 6 oder mehr Zellen der Winkel α zwischen den Drahtsegmenten oder Drähten und einer in Längsrichtung der Gitterstruktur verlaufenden Längsachse zumindest abschnittsweise mindestens 45°, insbesondere mindestens 50°, insbesondere mindestens 55°, insbesondere mindestens 60°, insbesondere mindestens 65°, insbesondere mindestens 70°, insbesondere mindestens 75° beträgt. Bei 12 oder mehr Zellen beträgt der Winkel α zwischen den Drahtsegmenten oder Drähten und einer in Längsrichtung der Gitterstruktur verlaufenden Längsachse zumindest abschnittsweise mindestens 45°, insbesondere mindestens 50°, insbesondere mindestens 55°, insbesondere mindestens 60°, insbesondere mindestens 65°, insbesondere mindestens 70°, insbesondere mindestens 75°. Bei 16 oder mehr Zellen beträgt der Winkel α zwischen den Drahtsegmenten oder Drähten und einer in Längsrichtung der Gitterstruktur verlaufenden Längsachse zumindest abschnittsweise mindestens 45°, insbesondere mindestens 50°, insbesondere mindestens 55°, insbesondere mindestens 60°, insbesondere mindestens 65°, insbesondere mindestens 70°, insbesondere mindestens 75°. Bei 24 oder mehr Zellen beträgt der Winkel α zwischen den Drahtsegmenten oder Drähten und einer in Längsrichtung der Gitterstruktur verlaufenden Längsachse zumindest abschnittsweise mindestens 45°, insbesondere mindestens 50°, insbesondere mindestens 55°, insbesondere mindestens 60°, insbesondere mindestens 65°, insbesondere mindestens 70°, insbesondere mindestens 75°. Bei 36 oder mehr Zellen, insbesondere bei 40 Zellen beträgt der Winkel α zwischen den Drahtsegmenten oder Drähten und einer in Längsrichtung der Gitterstruktur verlaufenden Längsachse zumindest abschnittsweise mindestens 45°, insbesondere mindestens 50°, insbesondere mindestens 55°, insbesondere mindestens 60°, insbesondere mindestens 65°, insbesondere mindestens 70°, insbesondere mindestens 75°. Bei 48 oder mehr Zellen beträgt der Winkel α zwischen den Drahtsegmenten oder Drähten und einer in Längsrichtung der Gitterstruktur verlaufenden Längsachse zumindest abschnittsweise mindestens 45°, insbesondere mindestens 50°, insbesondere mindestens 55°, insbesondere mindestens 60°, insbesondere mindestens 65°, insbesondere mindestens 70°, insbesondere mindestens 75°.

Der Drahtquerschnitt ist nicht auf eine bestimmte Form beschränkt. Möglich sind runde, insbesondere kreisrunde, elliptische, eckige oder andere Querschnittsformen des Drahtes. Das Funktionselement kann ein zumindest abschnittsweise, insbesondere vollständig röhrchenförmiges Geflecht, wie einen Stent bilden. Andere Anwendungen, wie Okkluder, Flow Diverter, Filter oder Thrombektomie Devices sind möglich.

Die Erfindung hat mehrere Vorteile.

Die auf der Drahtoberfläche gebildete Oxidschicht ist nickelarm und enthält TiO₂, wodurch das Korrosionsverhalten und die Biokompatibilität des Implantats verbessert werden. Die Ausbildung der Oxidschicht als Mischoxidschicht, in der wenigstens ein Nitrid vorhanden ist, insbesondere Titanitrid und/oder Titanoxinitrid, erhöht die Schichthärte, wodurch der Verschleiß bei Beanspruchung des Funktionselementes, insbesondere des Implantats, im Gefäß verringert wird. Dieser Vorteil kommt besonders bei Geflechten, wie bei geflochtenen Stents zum Tragen, die eine große Anzahl von Drähten mit vielen Kreuzungs- und Berührungspunkten aufweisen. Da es an jedem Kreuzungs- und Berührungspunkten durch die Relativbewegung der Drähte zueinander zu Reibung kommt, wirkt sich der verringerte Verschleiß auf Grund der harten und glatten Oberfläche der Drähte besonders stark aus.

Der verringerte Reibungskoeffizient der Oberfläche des Funktionselementes, insbesondere des Implantats führt zu einem verbesserten Gleitverhalten im Katheter. Die guten Gleiteigenschaften wirken einerseits zwischen den Drähten selbst, wodurch die Crimpbarkeit, d.h. die Fähigkeit des Funktionselementes komprimiert zu werden, verbessert wird. Damit lassen sich auch sehr feinmaschige Implantate mit einer großen Anzahl von Drähten, bspw. Flow Diverter, auf sehr kleine Durchmesser komprimieren, so dass bspw. Aneurysmen in engen Gefäßen mit dem Implantat erreicht und behandelt werden können.

Andererseits wirken die guten Gleiteigenschaften zwischen den Drähten und der Katheterinnenwand. Die dadurch verringerte Schiebekraft, die zum Bewegen des Funktionselements, insbesondere Implantats im Katheter erforderlich ist, erhöht die Sicherheit, da das Risiko des Blockierens und der Beschädigung des Funktionselements, insbesondere Implantats im Katheter verringert wird. Gleiches gilt für Zuführsysteme, bei denen die Zuführung des Funktionselements nicht durch Bewegung des Funktionselements selbst sondern durch eine Relativbewegung zwischen einem Teil des Zuführsystems und dem Funktionselement bewirkt wird.

Insgesamt wird die Qualität des Funktionselementes, insbesondere des Implantats verbessert, bspw. mit Blick auf die Compliance im Gefäß.

Die Schichtdicke von 15nm bis 100nm hat den Vorteil, dass die Abriebfestigkeit im Vergleich zu einem Draht, der nur elektropoliert ist, verbessert wird. Die natürliche Oxidschicht, die sich nach einer Elektropolitur einstellt, hat eine Schichtdicke von ca. 3-5nm, weshalb die Schicht leicht abgerieben werden kann. Die erfindungsgemäß erhöhte Schichtdicke zusammen mit der Nitridierung der Oxidschicht verbessert die Abriebfestigkeit.

Im Vergleich zu herkömmlichen Oxidschichten auf NiTi-Drähten mit einer Dicke von mehr als 200nm, die sich bei der Drahtherstellung einstellen, ist die erfindungsgemäße Mischoxidschicht schützender gegen den Austritt von Nickel-Ionen, was sich bspw. an dem guten Korrosionsverhalten der Schicht zeigt. Die Erfindung vereint daher die guten Schutzeigenschaften einer sehr dünnen Oxidschicht mit der guten Abriebfestigkeit einer relativ dicken Schicht sowie mit guten Gleiteigenschaften.

Vorteilhafterweise beträgt die Schichtdicke mindestens 30nm, insbesondere mindestens 35nm, insbesondere mindestens 40nm, insbesondere mindestens 45nm, insbesondere mindestens 50nm, insbesondere mindestens 55nm. Mit der Erhöhung der Untergrenze des Schichtdickenbereiches wird die Abriebfestigkeit weiter verbessert.

Wenn die Schichtdicke höchstens 95nm, insbesondere höchstens 90nm, insbesondere höchstens 85nm, insbesondere höchstens 80nm, insbesondere höchstens 75nm, insbesondere höchstens 70nm, insbesondere höchstens 65nm, insbesondere höchstens 60nm beträgt, werden durch die Verringerung der Obergrenze des Schichtdickenbereiches die schützenden Eigenschaften der Schicht verbessert. Außerdem sinkt das Risiko, dass sich - insbesondere bei Deformationen der Drähte - Schichtbestandteile von der Drahtoberfläche löst bzw. die Schicht brüchig wird.

Die vorstehend genannten Werte für die Untergrenze und die Obergrenze können jeweils miteinander kombiniert werden, um eine gezielte Verbesserung der jeweiligen Schichteigenschaften einzustellen. Bspw. kann die Untergrenze für die obigen Maximalwerte 30nm betragen. Ein besonders vorteilhafter Bereich der Schichtdicke beträgt von 20nm bis 90nm, insbesondere von 30nm bis 90nm, insbesondere von 40nm bis 80nm, insbesondere von 50nm bis 70nm.

Das Drahtgeflecht kann (im expandierten Zustand) einen Außendurchmesser von ≤ 7 mm, vorzugsweise ≤ 6 mm, noch weiter vorzugsweise ≤ 5 mm, noch weiter vorzugsweise ≤ 4,5 mm, noch weiter vorzugsweise ≤ 4,0 mm, noch weiter vorzugsweise ≤ 3,5 mm, aufweisen. Besonders bevorzugt ist eine Obergrenze des Durchmessers von 6 mm.

Mindestens ein Draht des Drahtgeflechts (vorzugsweise mehrere oder alle Drähte des Drahtgeflechtes) kann einen Durchmesser von ≤ 120 µm, ≤ 90 µm, vorzugsweise ≤ 76 µm, noch weiter vorzugsweise ≤ 51 µm, noch weiter vorzugsweise ≤ 40 µm, noch weiter vorzugsweise ≤ 35 µm, noch weiter vorzugsweise ≤ 25 µm, aufweisen. Die Untergrenze kann 20 µm betragen. Ein derartiger Draht bzw. ein derartiges Drahtgeflecht ist besonders einfach in der Handhabung und einfach im Körper des Patienten einzubringen. Unter dem jeweiligen "Durchmesser" wird (hier und im Folgenden) der maximale Durchmesser (in Radialrichtung) verstanden.

Am distalen Axialende kann das Drahtgeflecht geschlossene Endschlaufen aufweisen. Zur Bildung der Endschlaufe kommt der Draht aus dem Geflecht, wird umgelenkt und in das Geflecht zurückgeführt. Die distalen Endschlaufen verbessern das Schiebeverhalten im Katheter. Das proximale Axialende ist durch freie Drahtenden gebildet, die im Bereich der endnahen Überkreuzungen verbunden oder lose angeordnet sein können. Alternativ kann das Geflecht aus einem einzigen Einzeldraht gebildet sein, der am proximalen und distalen Ende geschlossene Endschlaufen bildet.

Das Funktionselement, insbesondere Implantat, insbesondere Stent, kann derart ausgebildet sein, dass eine Schiebekraftwirkung höchstens 1,5 N, vorzugsweise höchstens 1,3 N, vorzugsweise höchstens 1,1 N, vorzugsweise höchstens 0,9 N, vorzugsweise höchstens 0,8 N, vorzugsweise höchstens 0,7 N, vorzugsweise höchstens 0,6 N, vorzugsweise höchstens 0,5 N pro 10 mm Länge (in expandiertem Zustand des Implantats bzw. Stents) beträgt, bei einem Katheter-Innendurchmesser von ≤ 0,8 mm, vorzugsweise ≤ 0,7 mm, weiter vorzugsweise ≤ 0,6 mm, noch weiter vorzugsweise ≤ 0,5 mm, noch weiter vorzugsweise ≤ 0,4 mm.

Die Schiebekraft ist diejenige Kraft, die überwunden werden muss, um das Implantat, insbesondere den Stent, innerhalb eines Katheters bzw. Schlauches mit oben konkretisiertem Innendurchmesser zu bewegen. Die Kraftobergrenzen gelten insbesondere für ein Geflecht mit mindestens oder genau 40 Drähten und/oder Drahtsegmenten, insbesondere mit mindestens oder genau 48 Drähten und/oder Drahtsegmenten und einem Drahtdurchmesser von mindestens oder genau 35µm bzw. mindestens oder genau 40µm.

Der Drahtdurchmesser kann dabei mindestens oder genau 30µm, insbesondere mindestens oder genau 35µm, insbesondere mindestens oder genau 40µm, betragen. Der Gesamtdurchmesser des Implantats kann höchstens oder genau 6mm, insbesondere höchstens oder genau 5,5mm, insbesondere höchstens oder genau 5mm, insbesondere höchstens oder genau 4,5mm, insbesondere höchstens oder genau 4mm, betragen.

Das oben beschriebene Funktionselement, insbesondere Implantat kann Bestandteil eines Systems sein, umfassend das Funktionselement sowie einen Katheter, wobei ein Katheter-Innendurchmesser vorzugsweise ≤ 0,8 mm, weiter vorzugsweise ≤ 0,7 mm, noch weiter vorzugsweise ≤ 0,6 mm, noch weiter vorzugsweise ≤ 0,5 mm, noch weiter vorzugsweise ≤ 0,4 mm, ist. Unabhängig offenbart und beansprucht wird in diesem Zusammenhang auch die Verwendung eines wie oben beschrieben (hergestellten) Funktionselementes, insbesondere Implantates für einen Katheter und/oder als Stent.

Das Drahtgeflecht oder allgemein Drahtgebilde kann zumindest teilweise aus Kompositdrähten hergestellt werden, wobei ein innerer Kern des Drahtes aus einem röntgensichtbaren Material, wie Platin oder Tantal besteht.

Bei einer bevorzugten Ausführungsform bildet der Peak der Sauerstoffkonzentration in der Mischoxidschicht ein Plateau. Bspw. kann das Plateau in einer Schichttiefe von 5nm bis 50nm, insbesondere von 10nm bis 40nm, insbesondere von 15nm bis 30nm ausgebildet sein. Die Gesamtschichtdicke beträgt dabei mindestens ca. 60nm. Als Grund für das Plateau wird angenommen, dass der Sauerstoff sich nach Außen zur Schichtoberfläche hin bevorzugt mit Stickstoff und nach Innen zum Drahtmaterial hin mit Titan verbindet.

Das Verhältnis der Intensitäten zwischen Stickstoff und Sauerstoff (N/O) beträgt im Bereich des Sauerstoffplateaus höchstens 1:2,0, insbesondere von 1:2,5 bis 1:10, und nimmt zum Drahtmetallkörper hin ab, wobei die Intensität jeweils durch Augerelektronenspektroskopie (AES) im Tiefenprofile durch die Mischoxidschicht ermittelt wird. An der Schichtoberfläche beträgt das Verhältnis der Intensitäten zwischen Stickstoff und Sauerstoff (N/O) maximal ca. 1:2,0, insbesondere ca. 1:2,5. Das Verhältnis 1:2,0 liegt in der unmittelbaren Randschicht der Schichtaußenfläche (ca. 5nm-10nm), das Verhältnis 1:6 in etwa bei einer Schichttiefe von ca. 20-25nm und das Verhältnis 1:10 in etwa bei einer Schichttiefe von ca. 35-40 nm vor. Allgemein nimmt also das Verhältnis N/O zum Drahtmetallkörper hin ab.

Da Sauerstoff nicht im Drahtmaterial, sondern nur in der Schicht vorhanden ist, wird die Stickstoffintensität der Mischoxidschicht in Bezug auf die Sauerstoffintensität angegeben. Dadurch lässt sich indirekt der Stickstoffgehalt der Mischoxidschicht im Bereich der Schichtdicke charakterisieren.

AES wird bekanntermaßen zur Analyse der in einer oberflächennahen Schicht enthaltenen Elemente eines Materials eingesetzt. Durch sukzessiven Abtrag der Schicht durch Sputtern wird durch die AES-Analyse der jeweils freigelegten Schichtoberfläche ein Tiefenprofil der Elementverteilung in der Schicht erzeugt, das zur Charakterisierung des Stickstoffgehaltes bezogen auf den Sauerstoffgehalt sowie zum Nachweis des Konzentrationsverlaufs der übrigen Elemente, wie Ni und Ti herangezogen wird. Die gemessene Intensität des jeweiligen Elementes ergibt sich in bekannter Weise aus den bei der AES-Analyse durch Elektronenbeschuss emittierten Auger-Elektronen.

Vorzugsweise liegt der Stickstoff bis in eine Tiefe der Mischoxidschicht von bis zu 2/6, insbesondere bis zu 3/6, insbesondere bis zu 4/6 der Gesamtdicke der Mischoxidschicht einschließlich der ersten Oxidschicht vor. In absoluten Werten geht die stickstoffhaltige Randschicht bis in eine Schichttiefe von 10nm, insbesondere 20nm, insbesondere 30nm, insbesondere 40nm, insbesondere 50nm, bspw. bei einer Mischoxidschicht mit einer Gesamtdicke von ca. 60nm. Dadurch wird eine harte Randschicht der Mischoxidschicht erzeugt und das Abriebverhalten verbessert.

Zur Herstellung des intravaskulären Funktionselementes kommt ein Verfahren zum Einsatz, bei dem ein Metallkörper des Drahtes mit metallischer Oberfläche bereitgestellt wird. Dann wird eine erste Oxidschicht auf der metallischen Oberfläche des Metallkörpers ausgebildet. Zur thermischen Ausbildung einer zweiten Mischoxidschicht auf der ersten Oxidschicht wird eine Wärmebehandlung des Drahtes in einem Stickstoff enthaltenden Salzbad durchgeführt, wobei die Gesamtschichtdicke von 15nm bis 100nm beträgt und die Mischoxidschicht TiO2 und wenigstens ein Nitrid, insbesondere Titanoxinitrid und/oder Titanitrid aufweist.

Für die Bereitstellung des Drahtmetallkörpers mit metallischer Oberfläche wird eine Vorbehandlung durchgeführt, bei der eine üblicherweise auf der Drahtoberfläche vorhandene Oxidschicht entfernt wird. Diese Oxidschicht mit einer Dicke von 0,2µm bis 5µm entsteht bei der Drahtherstellung, wenn zur Einstellung der Materialeigenschaften des Drahtes eine Wärmebehandlung durchgeführt wird. Für die Entfernung der Oxidschicht kommen verschiedene Verfahren in Frage, die im Rahmen der Erfindung eingesetzt werden können, um einen Drahtmetallkörper mit metallischer Oberfläche bereitszustellen. Die Erfindung ist nicht auf diese Verfahren beschränkt.

Bei einer bevorzugten Ausführung wird die obige herstellungsbedingte Oxidschicht durch Elektropolieren entfernt. Dabei wird in an sich bekannter Weise in einem Elektrolytbad unter Einwirkung von Strom von der Drahtoberfläche Metall zusammen mit Verunreinigungen sowie einer auf der Drahtoberfläche natürlich gebildeten Oxidschicht abgetragen, so dass nach dem Elektropolieren eine glatte und homogene metallische Oberfläche vorliegt, die im Wesentlichen oxidfrei ist.

Der Drahtmetallkörper mit metallischer Oberfläche kann auf andere Weise erzeugt werden, bspw. durch chemisches oder elektrochemisches oder mechanisches Abtragen der Oberflächenschicht des Drahtes. Eine Möglichkeit des chemischen Abtragens ist das Ätzen oder Beizen. Dabei wird die natürliche Oxidschicht entfernt, so dass die metallische Oberfläche des Drahtkörpers freiliegt. Die hierfür verwendeten Prozessparameter sind dem Fachmann bspw. aus US2004/0117001 A1 bekannt. Außerdem sind mikroabrasive Antragsverfahren bekannt, die ebenfalls bei der Erfindung eingesetzt werden können.

Auf der metallischen Oberfläche des Drahtmetallkörpers wird eine erste Oxidschicht ausgebildet, auf die die zweite Mischoxidschicht thermisch aufgebracht wird. Die Bildung der ersten Oxidschicht kann im einfachsten Fall in Form einer natürlichen Oxidschicht erfolgen, die entsteht, wenn die metallische Oberfläche des Drahtmetallkörpers der Umgebungsluft ausgesetzt wird. Wenn die Vorbehandlung des Drahtes bspw. durch Elektropolieren erfolgt, hat sich gezeigt, dass die natürliche, erste Oxidschicht eine Dicke von ca. 3nm bis 10nm aufweist.

Vorzugsweise wird der Draht zur Herstellung eines Drahtgebildes, d.h. der Gitterstruktur, vor der Wärmebehandlung verformt, wobei sich wenigstens ein Abschnitt, insbesondere mehrere Abschnitte des verformten Drahtes überkreuzen und/oder berühren. Das Drahtgebilde kann bspw. ein Geflecht, konkret ein geflochtenes Implantat, bspw. ein geflochtener Stent, ein Graft-Stent, ein geflochtenes Okklusionsdevice oder ein geflochtener Flow Diverter umfassen. Für das Geflecht werden in bekannter Weise Maschen gebildet, die durch Drahtüberkreuzungen begrenzt sind. Das Geflecht kann aus einem einzigen Draht oder aus mehreren Drähten gebildet sein. Dabei können herkömmliche Flechttechniken verwendet werden. Im Bereich der Drahtüberkreuzungen können sich die Drähte bzw. Abschnitte berühren oder voneinander beabstandet sein.

Die bevorzugte Ausbildung des Drahtgebildes nach dem Elektropolieren des Drahtes bzw. allgemein vor der Wärmebehandlung zur Bildung der Mischoxidschicht hat den Vorteil, dass der Draht in der für das Elektropolieren eingesetzten Lösung im geraden Zustand, d.h. ohne Drahtüberkreuzung oder Kontaktstelle, behandelt wird. Damit wird erreicht, dass die Lösung die Drahtoberfläche gleichmäßig im gesamten Oberflächenbereich benetzt. Eine Abschattung im Bereich von Drahtüberkreuzungen und/oder Kontaktstellen wird vermieden, da die Ausbildung des Drahtgebildes erst nach dem Elektropolieren erfolgt. Durch den dadurch erreichbaren gleichmäßigen Abtrag beim Elektropolieren wird die Voraussetzung für die Ausbildung einer homogenen und möglichst konstant dicken Oxidschicht in den nachfolgenden Prozessschritten geschaffen. Die Gitterstruktur bzw. das Geflecht zeichnet sich daher dadurch aus, dass der Durchmesser entlang des Drahtes auch im Bereich jeder Überkreuzung konstant bzw. nahezu konstant bleibt.

Konkret wird durch das Elektropolieren die nach der Drahtherstellung vorhandene Oxidschicht samt Verunreinigungen entfernt, so dass nach dem Elektropolieren zunächst eine im wesentlichen blanke Metalloberfläche des Drahtes vorliegt. Der Draht wird aus der Lösung entfernt und der Umgebungsluft ausgesetzt. Durch den Kontakt mit Luft bildet sich eine natürliche Oxidschicht mit einer Dicke von ca. 5nm an der Drahtoberfläche. Diese Oxidschicht ist homogen und weist eine im Wesentlichen konstante Dicke auf. Sie besteht hauptsächlich aus TiO₂. Der Ni-Gehalt in der Oxidschicht nimmt zur Oberfläche hin stark ab, die im Wesentlichen nickelfrei ist.

Nach dem Umformen des Drahtes zu einem Drahtgebilde mit wenigstens einer Überkreuzung erfolgt die thermische Oxidierung im Salzbad. Dadurch wird die Oberfläche nach dem Elektropolieren modifiziert, insbesondere passiviert und gehärtet. Da die nach dem Elektropolieren natürlich ausgebildete Oxidschicht zumindest im oberflächennahen Grenzbereich nickelarm oder sogar nickelfrei ist und somit wie eine Barriere zur Metallgrenzfläche des Drahtes wirkt, enthält die thermische ausgebildete Oxidschicht ebenfalls nur einen geringen Ni-Gehalt bzw. ist zumindest im oberflächennahen Grenzbereich nickelarm oder sogar nickelfrei. Durch die sich anschließende thermische Behandlung im stickstoffhaltigen Salzbad wird auf der natürlich ausgebildeten Oxidschicht eine dichte Mischoxidschicht erzeugt, die TiO2 enthält. Zusätzlich enthält die Mischoxidschicht Anteile an Stickstoff, welcher als Titanoxinitrid und/oder Titanitrid gebunden ist. Titanoxinitrid und/oder Titanitrid ergibt sich aus dem Salzbad, bspw. bei einer Verwendung eines Alkalimetall-Stickstoff-Salzes, insbesondere Kaliumnitrat oder Natriumnitrit oder einem Gemisch aus Kaliumnitrat und Natriumnitrit, ergibt. Das thermisch ausgeformte Nitrid, insbesondere Titanoxinitrid und/oder Titaninitrid, wirkt als Hartstoff, der die Schichthärte erhöht und das Verschleiß- und Reibungsverhalten des Funktionselemetes, insbesondere Implantats verbessert.

Im Gegensatz zum Stand der Technik wird bei dieser Ausführung also zuerst eine Elektropolitur durchgeführt und anschließend eine Wärmebehandlung im Salzbad. Insgesamt wird durch die thermische (inerte) Salzbadbehandlung eine Oxidierung im Anschluss an den elektrochemischen Polierprozess realisiert. Dadurch sind sehr dichte, sowie reibungsarme und verschleißfeste Oxidschichten, die dicker als 10 nm sein können, herstellbar. Außerdem können besonders günstige physikalische, beispielsweise hinsichtlich der Radialkraft und des Ermüdungsverhaltens, physikalisch-chemische, insbesondere bezüglich der Nickel-Freisetzung und des Korrosionsverhaltens, und biologische Grenzflächencharakteristika, beispielsweise die Thrombogenität, von Funktionselementen, insbesondere Implantaten wie Stents, vergleichsweise genau und einfach eingestellt werden. Dadurch lässt sich das Oberflächenverhalten und die Biokompatibilität signifikant verbessern.

Die Erfindung ist nicht auf eine spezielle NiTi-Legierung eingeschränkt, sondern allgemein bei den in der Medizintechnik gebräuchlichen NiTi-Legierungen einsetzbar, aus denen intravaskuläre Funktionselemente, insbesondere Implantate hergestellt werden, deren Oberflächen durch eine Oxidschicht geschützt werden soll.

Als Legierung kommen bspw. verschiedene binäre Verbindungen auf Ni-Basis in Frage, wie bspw. NiTi Legierungen, insbesondere Nitinol (Ni 55 Gew.-%, Ti 45 Gew.-%), oder verschiedene ternäre Verbindungen wie bspw. NiTiFe oder NiTiNb oder NiTiCr oder quarternäre Legierungen wie NiTiCoCr.

Der Draht kann mindestens 5 Gew.-%, vorzugsweise mindestens 10 Gew.-%, vorzugsweise mindestens 20 Gew.-%, vorzugsweise mindestens 40 Gew.-%, Nickel aufweisen. Der Draht kann weiterhin vorzugsweise höchstens 80 Gew.-%, vorzugsweise höchstens 60 Gew.-%, vorzugsweise höchstens 55 Gew.-% vorzugsweise höchstens 50 Gew.-% Nickel aufweisen. Der Titangehalt kann vorzugsweise mindestens 10 Gew.-%, vorzugsweise mindestens 30 Gew.-%, vorzugsweise mindestens 40 Gew.-%, vorzugsweise mindestens 50 Gew.-%, betragen. Eine Obergrenze für den Titangehalt kann 90 Gew.-%, vorzugsweise 80 Gew.-%, vorzugsweise 65 Gew.-%, vorzugsweise 60 Gew.-%, vorzugsweise 55 Gew.-%, betragen.

Vorzugsweise erfolgt die Wärmebeaufschlagung (zumindest teilweise) gleichzeitig mit dem Eintauchen in das Salzbad, weiter vorzugsweise erfolgt zumindest während 10%, weiter vorzugsweise zumindest während 30%, noch weiter vorzugsweise zumindest 50%, noch weiter bevorzugt zumindest 90% der Salzbad-Eintauchzeit eine Wärmebeaufschlagung. Vor oder nach dem Eintauchen in das Salzbad kann eine zusätzliche Wärmebehandlung erfolgen. Bevorzugt erfolgt jedoch nur während des Eintauchens in das Salzbad eine Wärmebehandlung.

Bei einer Ausführung erfolgt die Wärmebehandlung im Salzbad in wenigstens zwei Schritten, wobei der zweite Schritt nach dem Einbringen von Funktionsmitteln, wie röntgensichtbaren Markierungen, und/oder nach Fügeprozessen erfolgt.. Es hat sich gezeigt, dass bei einer Beschädigung der Mischoxidschicht durch mechanische oder thermische Fügeprozesse wie die Befestigung von röntgensichtbaren Materialien eine zweite Wärmehandlung diese Beschädigungen heilen kann.

Die Behandlungszeit, insbesondere des ersten Wärmebehandlungsschrittes beträgt vorzugsweise mindestens 1 min, vorzugsweise mindestens 2 min und/oder vorzugsweise höchstens 8 min, vorzugsweise höchstens 7 min, vorzugsweise höchstens 6 min, vorzugsweise höchstens 5 min, vorzugsweise höchstens 4 min, vorzugsweise höchstens 3 min. Die Dauer des zweiten Wärmebehandlungsschrittes kann mindestens 50% kürzer als die Dauer des ersten Wärmebehandlungsschrittes sein. Vorzugsweise beträgt die Dauer des ersten Wärmebehandlungsschrittes ca. 2min bis 4min und die Dauer des nachfolgenden zweiten Wärmebehandlungsschrittes ca. 20s bis 60s.

Die Temperaturobergrenze der Wärmebehandlung im Salzbad beträgt vorzugsweise 550°C, insbesondere 540°C, insbesondere 530°C, insbesondere 520°C. Die Untergrenze kann 400°C, insbesondere 420°C, insbesondere 440°C, insbesondere 460°C, insbesondere 480°C betragen.

Das Implantat ist vorzugsweise ein geflochtener Stent, kann jedoch auch ein anderes Implantat, beispielsweise ein Flow-Diverter oder ein Stent-Graft oder ein intravaskuläres Verschlussdevice oder ein intravaskuläres Coil sein.

Der Nickel-Anteil in der Mischoxidschicht beträgt vorzugsweise weniger als 6 Gew.-%, noch weiter vorzugsweise weniger als 3 Gew.-%, noch weiter vorzugsweise weniger als 2 Gew.-% jeweils wenigstens bis in eine Schichttiefe von 30% der Gesamtdicke der Mischoxidschicht, ausgehend von der Schichtoberfläche, insbesondere bis in eine Schichttiefe von 50% der Gesamtdicke der Mischoxidschicht, ausgehend von der Schichtoberfläche. Konkret kann sich der nickelarme Schichtbereich bis in eine Tiefe von 20nm - 40nm bei einer Schichtdicke von 60nm bis 100nm erstrecken. Dabei kann die Oberfläche der Mischoxidschicht überwiegend aus TiO2 bestehen.

Ein Kontaktwinkel, d.h. der Benetzungswinkel der Oberfläche des Implantats beträgt bei Benetzung mit destilliertem Wasser vorzugsweise weniger als 90°, weiter bevorzugt weniger als 80°, noch weiter vorzugsweise weniger als 75°, und/oder mindestens 30%, vorzugsweise mindestens 60°. Bei einem derartigen Kontaktwinkel (Benetzungswinkel) ist die Biokompatibilität des Implantats vergleichsweise hoch. Weiterhin kann ein derartiger Kontaktwinkel besonders einfach durch die weiter oben beschriebene Reihenfolge der Herstellungsverfahrensschritte eingestellt werden.

In einer weiteren Ausführungsform ist ein Salz des Salzbades ein Alkalimetall-Stickstoff-Salz, vorzugsweise Kalium-Stickstoff-Salz und/oder Natrium-Stickstoff-Salz, insbesondere Kaliumnitrat und/oder Natriumnitrit, insbesondere ein Gemisch aus Kaliumnitrat und Natriumnitrit. Weitere Salze können (müssen aber nicht) enthalten sein. Es hat sich gezeigt, dass sich durch ein derartiges nitrat- und/oder nitrithaltiges Salzbad eine Mischoxidschicht, insbesondere enthaltend Titanoxinitrid, realisieren lässt, die sich durch günstige Oberflächeneigenschaften (geringe Rauheit) und eine hohe Biokompatibilität auszeichnet.

Es hat sich gezeigt, dass dichte und schützende Mischoxidschichten erzielt werden, was sich anhand des verbesserten Korrosionsverhaltens der Implantate zeigt, wenn der Anteil an Kaliumnitrat größer als der Anteil an Natriumnitrit ist. Konkret kann der das Salzbad folgende Bestandteile enthalten:
30-40 Gew.-% KNO₃
25-35 Gew.-% NaNO₂
Rest übliche Kohlenstoffverbindungen und Verunreinigungen, wobei die Bedingung gilt, dass der Anteil an Kaliumnitrat größer als der Anteil an Natriumnitrit ist. Bei den Kohlenstoffverbindungen handelt es sich um Verbindungen, die dem Fachmann im Zusammenhang mit Salzbädern für die Wärmebehandlung bekannt sind. Vorzugsweise beträgt der Anteil an Kaliumnitrat 32-38 Gew.-%, insbesondere 34-36 Gew.-%. Der Anteil an Natriumnitrit kann 26-33 Gew.-%, insbesondere 27-30 Gew.-% .

Man nimmt an, dass der erhöhte Anteil an KNO₃, das eine höhere Zersetzungstemperatur als NaNO₂ aufweist, die Stickstoffanreicherung und Oxidierung begünstigt.

Die Mischoxidschicht kann mindestens 10 Gew.-%, weiter vorzugsweise mindestens 20 Gew.-%, weiter vorzugsweise mindestens 30 Gew.-%, weiter vorzugsweise mindestens 40 Gew.-%, weiter vorzugsweise mindestens 50 Gew.%. Titanoxinitrid und/oder Titanitrid aufweisen.

Bei dem Draht kann es sich um einen Endlosdraht handeln, der beispielsweise auf eine Spule und/oder von einer Spule gewickelt wird. Dadurch wird sowohl das Herstellungsverfahren - insbesondere des Elektropolierens - vereinfacht als auch ein einfach zu handhabendes Implantat realisiert.

Besonders bevorzugt ist es, wenn das Geflecht erst nach dem Elektropolieren bzw. allgemein vor der Wärmebehandlung gebildet wird. Bei dieser besonderen Ausführungsform wurde erkannt, dass im Stand der Technik üblicherweise die Kreuzungspunkte von (an sich bekannten) Drahtgeflechten eine Schwachstelle darstellen, da das Medium für die Elektropolitur in diesem Bereich der Überlappung der Drähte auf Grund von Abschattungseffekten nicht ausreichend wirken kann. Dadurch, dass erst nach dem Elektropolieren ein Geflecht gebildet wird, ist die Oberfläche des Drahtgeflechts insgesamt homogener und von vergleichsweise guter Biokompatibilität. Wenn der Draht bzw. die Drähte vor dem Flechten elektropoliert werden, wird ein vergleichsweise gleichmäßiger Drahtdurchmesser erreicht, was die Mechanik des Funktionselementes, insbesondere Implantats und die Compliance positiv beeinflusst. Würde die Elektropolitur erst nach dem Flechten erfolgen, käme es im Bereich von Überkreuzungen der Drähte, in denen die Elektropolitur-Lösung nicht ausreichend gut in Kontakt mit den Drähten ist, zu einer Reduzierung des Abtrags beim Elektropolieren. Dies würde zu unregelmäßigen Drahtdurchmessern bzw ungleichen Ausbildung der Oberflächeneingenschaften führen.

Vorzugsweise hat die Oberflächenschicht einen Nickel-Anteil von weniger als 6 Gew.-%. Vorzugsweise hat die Oberflächenschicht einen Nickel-Anteil von weniger als 3 Gew.-%, noch weiter vorzugsweise von weniger als 2 Gew.-%.

Insgesamt wird durch die oben beschriebenen Maßnahmen insbesondere die Nickel-Freisetzung von Implantaten minimiert, die Radialkraft von medizinischen Implantaten (z.B. aus Nitinol) optimiert, das Werkstoff-Ermüdungsverhalten des Implantates (z.B. aus Nitinol) verbessert, das Korrosionsverhalten des Implantates (z.B. aus Nitinol) verbessert, die Oberflächenrauheit des Implantats (z.B. aus Nitinol) verringert sowie die Thrombogenität von medizinischen Implantaten (z.B. aus Nitinol) verbessert. Außerdem kommt es (aufgrund einer geringen bzw. nicht vorhandenen Porosität) zu einer äußerst geringen Ablagerung von Proteinen und weiteren Blutbestandteilen, die zur Bildung von Thromben führen kann. An Stelle von Geflechten können allgemein Drahtgebilde zum Einsatz kommen, die mindestens eine Kontaktstelle zwischen zwei Drähten haben (Vorrichtungen z.B., die aus Wire-Forming hergestellt werden, oder anderen Textilverfahren).

Sollte ein Parameter (z.B. die Schichtdicke) variieren, kann der jeweilige Maximalwert oder der (geometrische) Mitteilwert gemeint sein.

Die Erfindung wird im Folgenden anhand von Ausführungsbeispielen unter Bezugnahme auf die beigefügten, schematischen Zeichnungen näher erläutert. Darin zeigen:
- Fig. 1: mehrere Drähte in einem Elektropolitur-Bad;
- Fig. 2: ein schematisches Geflecht aus mehreren Drähten;
- Fig. 3: das Geflecht gemäß Fig. 2 in einem Salzbad;
- Fig. 4: ein Tiefenprofil einer Mischoxidschicht eines Funktionselementes nach einem erfindungsgemäßen Ausführungsbeispiel mit einer Schichtdicke von ca. 60nm (Probel.6pro);
- Fig. 5: ein Tiefenprofil einer Mischoxidschicht eines Funktionselementes nach einem Vergleichsbeispiel mit einer Schichtdicke von ca. 220nm (Auger2.5+3.pro);
- Fig. 6: eine Korrosionskurve (0611-170-01) eines Funktionselementes als Vergleichsbeispiel, bei dem ein nicht-elektropolierter Draht verwendet wird ;
- Fig. 7: eine Korrosionskurve (1586-170-04) eines Funktionselementes nach einem erfindungsgemäßen Ausführungsbeispiel ;
- Fig. 8: eine REM-Aufnahme einer unbehandelten Vergleichsprobe und
- Fig. 9: eine REM-Aufnahme einer Drahtoberfläche, die nach einer Ausführung des erfindungsgemäßen Verfahrens erzeugt ist.

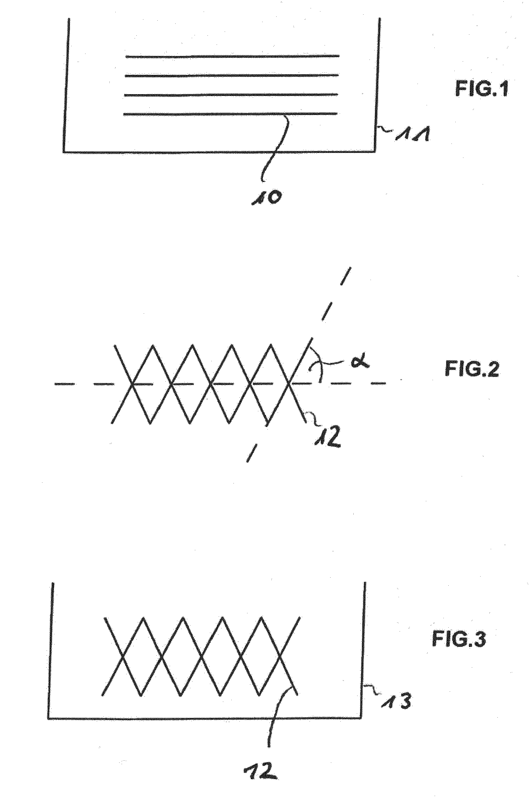

Fig. 1 zeigt einen ersten Schritt für die Herstellung eines Stents. Mehrere Drähte 10 (in der schematischen Zeichnung vier Drähte) werden in ein Elektropolitur-Bad 11 eines Elektrolyten eingetaucht. Dieser Schritt kann wie in der US 2004/0117001 A1, abgesehen von der zeitlichen Reihenfolge, durchgeführt werden.

Fig. 2 zeigt (schematisch) ein Geflecht 12 aus den Drähten 10. Das Geflecht 12 ist dabei im aufgeklappten Zustand dargestellt, so dass die gesamte Umfangsfläche des Geflechts 12 in der Zeichnungsebene abgebildet ist. Nach dem Schritt des Flechtens wird das Geflecht 12 in ein Salzbad 13 eingetaucht und wärmebehandelt (sh. Fig. 3). In dem Salzbad 13 erhält das Geflecht 12 bzw. der Stent seine endgültige Struktur einschließlich der passivierten Oberfläche. Dies schließt nicht aus, dass noch weitere Bearbeitungsschritte nachfolgen können.

### Beispiel

Die Erfindung wird beispielhaft anhand eines Funktionselements erläutert, das aus einer binären NiTi-Legierung, wie Nitinol, hergestellt ist. Andere NiTi enthaltende Legierung sind möglich. Dabei wird die Modifikation der Oberfläche durch die thermische Behandlung im Salzbad dargestellt, die für die Einstellung der Stickstoffkonzentration in der TiO2 Mischoxidschicht verantwortlich ist. Das Grundelement des Funktionselementes, nämlich der Draht, wird im ersten Schritt elektropoliert. Die Elektropolitur kann wie im Stand der Technik üblich durchgeführt werden, bspw. bei einer Temperatur T < 20° unter Verwendung einer Methanol-Schwefelsäure-Lösung. Auf dem elektropolierten Draht bildet sich durch Kontakt mit der Umgebungsluft spontan eine homogene natürliche Oxidschicht mit einer Schichtdicke von ca. 5nm.

Im zweiten Schritt wird aus dem elektropolierten Draht das Funktionselement, ein Stent, geflochten.

Im dritten Schritt wird das Funktionselement zur Erhöhung der Schichtdicke im Salzbad wärmebehandelt. Dabei wird eine Salzbadzusammensetzung verwendet, die aus folgenden Komponenten besteht:
ca. 35-36 Gew.-% KNO₃
ca. 27-29 Gew.-% NaNO₂
Rest übliche Kohlenstoffverbindungen und Verunreinigungen.

Es hat sich gezeigt, dass gute Ergebnisse erzielt werden können, wenn der Anteil an Kaliumnitrat größer als der Anteil an Natriumnitrit im Salzbad ist.

Die Prozesstemperaturen betragen ca. 490° C - 510° C . Im ersten Behandlungsschritt wird das Funktionselement ca. 2 bis 3min in das Salzbad getaucht. Dabei erfolgt die Ausbildung der Oxidschicht. Die Behandlungsdauer im zweiten Schritt beträgt ca. 30sec. oder weniger.

### Messverfahren

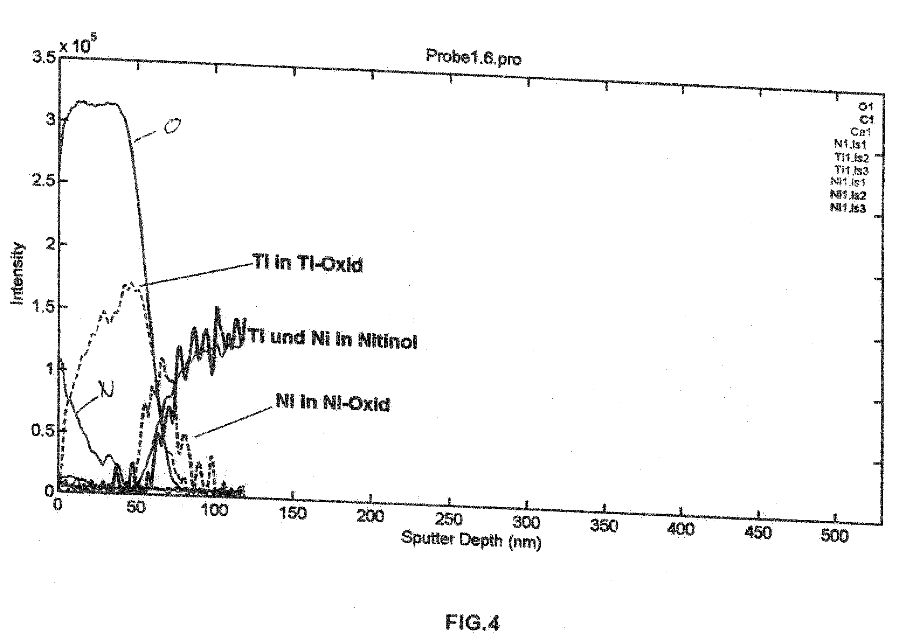

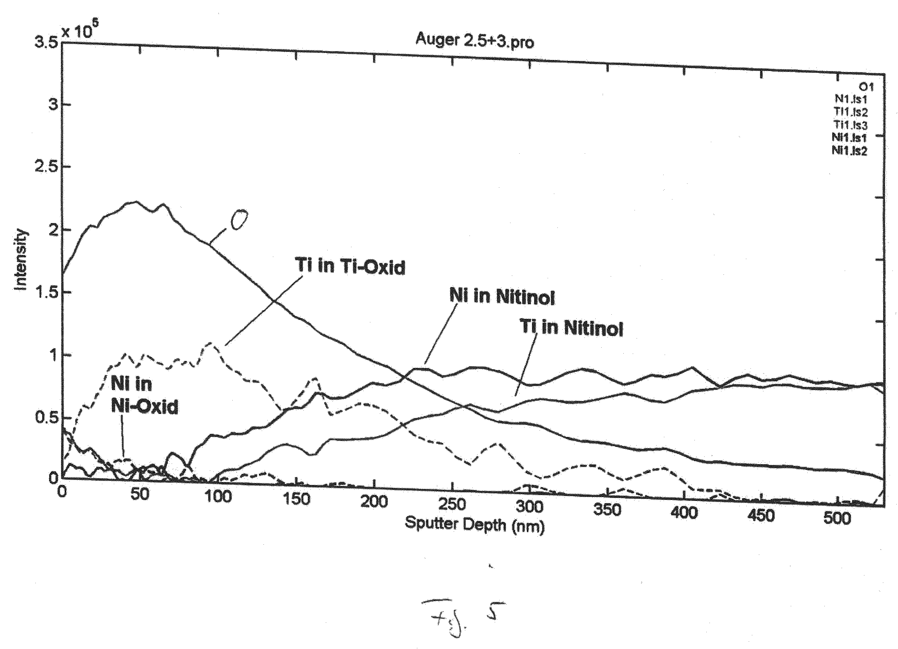

Die Messungen zur Erstellung des AES-Tiefenprofils gemäß Fig. 4, 5 wurden mit folgenden Parametern durchgeführt:
Primärelektronenenergie (Anregung): 5keV
Strahlstrom: 20nA
Elektronenstrahlraster (analysierter Bereich): 20µm x 20µm
Ionenstrahlenergie: 3keV (Fig. 4, Probe 1.6.pro, jeweils behandelt)
Strahlstrom: 2µA
Abtragrate: 59,3nm/min.
Ionenstrahlraster: 0,8mm x 0,8mm
Ionenstrahlenergie: 1keV (Fig. 5, Auger2.5+3.pro, unbehandelt)
Strahlstrom: 0,5µA
Abtragrate: 8,24nm/min.
Ionenstrahlraster: 0,8mm x 0,8mm
Probenwinkel (Elektronenstrahl - Probennormale): 30°

Für die Bestimmung der Intensitäten wurden folgende Elementpeaks verwendet:
Ti1: Ti LMM bei 390 eV
Ti2: Ti LMM bei 421 eV
N1: N KLL bei 389 eV
Ni1: Ni LMM bei 849 eV
O1: O KLL bei 510 eV

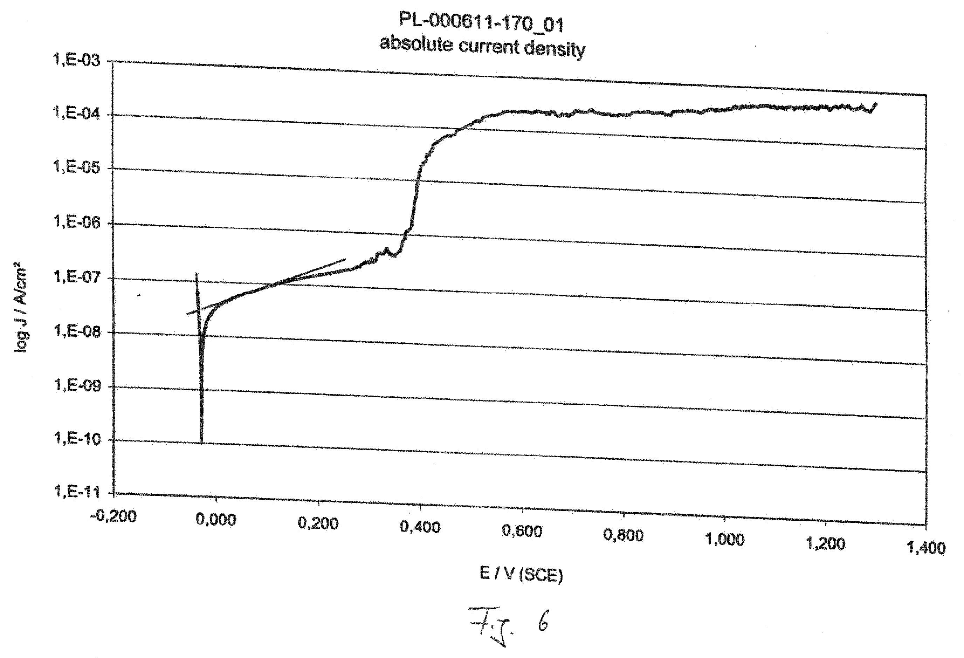

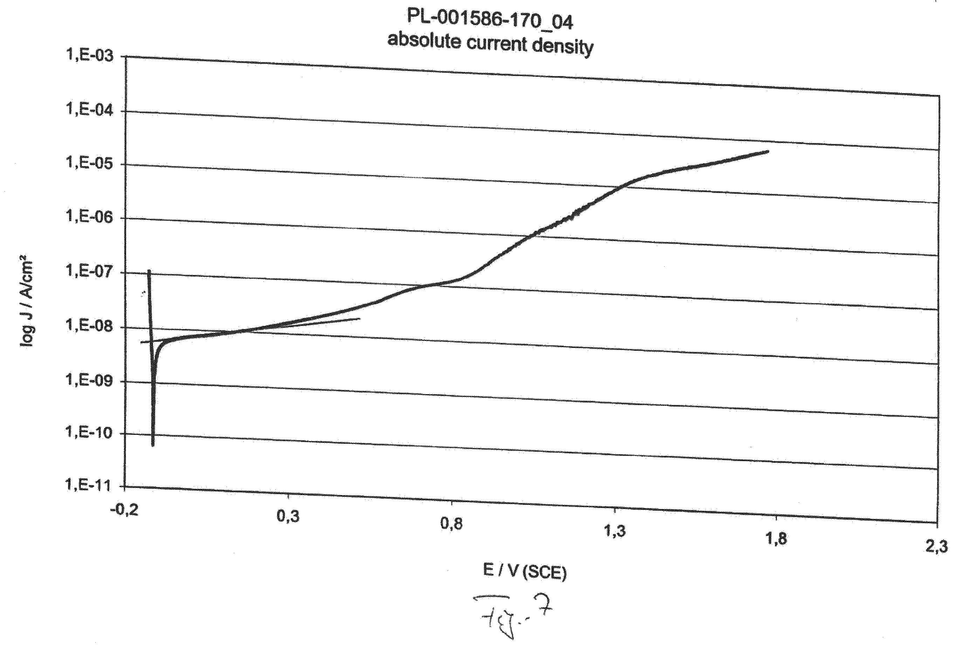

Die Messungen für die Korrosionskurven gemäß Fig. 6, 7 wurden nach ASTM F2129 "Standard Test Method for Conducting Cyclic Potentiodynamic Polarization Measurements to Determine the Corrosion Susceptibility of Small Implant Devices" durchgeführt.

### Ergebnisse und Diskussion

Das Tiefenprofil gemäß Fig. 4, bei der die Sputtertiefe auf 500 nm normiert ist, zeigt den Konzentrationsverlauf der Schichtelemente, der sich nach der Durchführung des vorstehend erläuterten Verfahrens ergibt. Dabei wird generell die Schichtdicke aus den Sputterparametern bestimmt. Alternativ zur Bestimmung der Schichtdicke mit 50% vom "Peak-Wert" TiO2 gerechnet werden. Demnach hat die Schicht eine Dicke von ca. 60nm, was sich aus der Schnittstelle des SauerstoffPeaks und der Peaks für metallisches Ti und Ni ergibt. Markiert sind in Fig. 4 die folgenden Peaks:
Sauerstoff
Stickstoff
Ti im Titanoxid
Titan (metallisches Titan)
Ni im Ni-Oxid
Ni

Besonders prägnant ist die Ausbildung des Sauerstoffpeaks als ein Plateau. Das Plateau befindet sich in einer Schichttiefe zwischen etwa 10nm und 40nm. Eine mögliche Erklärung wird darin gesehen, dass der Sauerstoff zum äusseren Teil der Schicht hin sich auch mit Stickstoff verbindet und zum inneren Teil der Schicht hin sich dann mit Titan verbindet. Der Stickstoff ist als chemische Verbindung in die Schicht eingebaut, und zwar als Titanoxinitrid. Das ergibt sich aus dem Verlauf des Sauerstoffsignals, das ein Plateau bildet. Ggf. ist der Stickstoff zusätzlich auch noch als Titanitrid vorhanden. Allgemein bedeutet der Plateauverlauf, dass die Sauerstoffintensität bereichsweise, insbesondere über eine Schichttiefe von mindestens 10nm konstant ist.

Das Verhältnis der Intensitäten zwischen Stickstoff und Sauerstoff N/O in der Schicht beträgt im Bereich des Sauerstoffplateaus in der größten Ausprägung ca. 1:3; 1:6; 1:10, wobei das Verhältnis 1:3 in der unmittelbaren Randschicht der Schichtaußenfläche (ca. 5nm-10nm ), das Verhältnis 1:6 in etwa bei einer Schichttiefe von ca. 20-25nm und das Verhältnis 1:10 in etwa bei einer Schichttiefe von ca. 35-40 nm vorliegt. An der Schichtoberfläche beträgt das Verhältnis ca. 1:2,5.

Aus Fig. 4 ergibt sich ferner, dass es im inneren Teil der Schicht, also nah am metallischen Drahtkörper, eine deutliche Anreicherung von Ni-Oxid gibt. Der Rest der Schicht weist kaum Nickel auf. Insbesondere die äußere Randschicht ist nickelarm. Dieser Konzentrationsverlauf könnte durch den Stickstoff bedingt sein, der sich ggf. bevorzugt gegenüber Ni mit dem Sauerstoff verbindet und somit eine Nickel-Anreicherung im äußeren Teil der Schicht unterbindet.

Fig. 5 zeigt das Tiefenprofil einer unbehandelten Probe mit einer Oxidschichtdicke von ca. 220nm (s. Schnittpunkt Ni/O). Die untersuchte Oxidschicht wurde durch die Wärmebehandlung bei der Drahtherstellung gebildet. Bei der Probe gemäß Fig. 5 wurde keine Vorbehandlung durchgeführt, d.h. die herstellungsbedingte Oxidschicht wurde auf der Oberfläche des Drahtes belassen. Fig. 5 zeigt, dass der Sauerstoffverlauf kein Plateau bildet. Die Sauerstoffintensität nimmt bis ca. 50nm zu und fällt dann ab. Im Unterschied zur Schicht gemäß Fig. 4 ist außerdem eine leichte Nickel-Oxid-Anreicherung im oberflächennahen Bereich der Schicht festzustellen. Die Nickel-Oxidanreicherung im metallkörpernahen Bereich der Schicht fehlt. Die Stickstoffintensität in der Schicht ist insgesamt deutlich kleiner als bei der Schicht gemäß Fig. 4.

Die Bewertung des Schutzverhaltens der Schicht erfolgt anhand der Korrosionskurven gemäß Fig. 6, 7, aus denen das elektrochemische Verhalten der Schichten und damit die interessierenden Schichteigenschaften, wie zB das Freisetzen von Nickel-Ionen, abgeleitet werden können. In den Fig. 6, 7 ist die Stromdichte J / A/cm² über der Spannung E/V (SCE) aufgetragen.

Fig. 7 zeigt die Korrosionskurve (1586-170-04) einer erfindungsgemäß hergestellten Schicht, die eine sehr niedrige Korrosionsstromdichte (<1x10-8 A/cm2) aufweist. Dies bedeutet, dass die Schicht eine geringe Durchlässigkeit für Metallionen und damit gute Schutzwirkung zeigt. Besonders wichtig ist, wie sich aus dem nahezu linearen Anstieg ergibt, dass kein Durchbruch, dh keine Pitting Korrosion auftritt. Die Schichteigenschaften sind demnach hervorragend.

Im Unterschied dazu liegt gemäß Fig. 6 der Korrosionsstrom bei der herkömmlich hergestellten Schicht über 1x10-7 A/cm². Bei etwa 400mV sind Durchbrüche zu beobachten, die auf den Beginn von Pitting-Korrosion, also Lochfraß hindeuten.

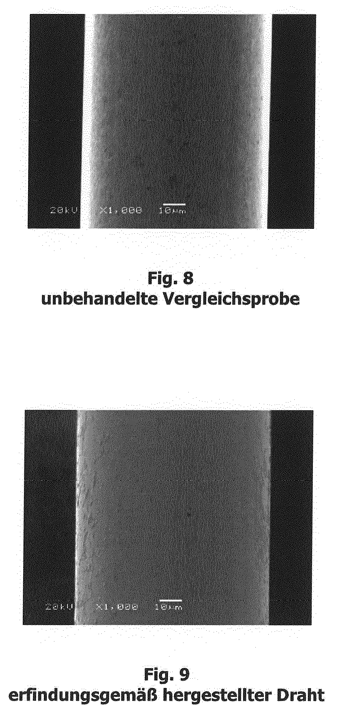

Die guten Oberflächeneigenschaften ergeben sich aus dem Vergleich zwischen der in Fig. 8 dargestellten Oberfläche eines unbehandelten Drahtes mit einer herstellungsbedingten Oxidschicht nd der in Fig. 9 gezeigten Oberfläche eines nach der Erfindung wärmebehandelten Drahtes. Die Oxidschicht des Drahtes gemäß Fig. 9 ist gleichmäßig dicht und porenfrei.

Mit dem erfindungsgemäßen Verfahren lassen sich sehr korrosionsstabile und feste Mischoxidschichten herstellen, die eine gute Schutzwirkung entfalten und sicher vor Abrieb schützen.

### Bezugszeichenliste

- α: Flechtwinkel
- 10: Draht
- 11: Elektropolitur-Bad
- 12: Geflecht
- 13: Salzbad

## Patentansprüche

1. Intravaskuläres Funktionselement mit einer röhrchenförmigen Gitterstruktur (12), die einen Draht (10) oder mehrere Drähte (10) jeweils aus einer Legierung mit den Legierungselementen Nickel und Titan aufweist, wobei eine Mischoxidschicht auf der Oberfläche des Drahtes (10) / der Drähte (10) mit einer Schichtdicke von 15nm bis 100nm ausgebildet ist, die TiO2 und wenigstens ein Nitrid, insbesondere Titanoxinitrid und/oder Titanitrid aufweist, wobei die Gitterstruktur in Längsrichtung und in Umfangsrichtung jeweils Zellen aus sich kreuzenden Drähten (10) oder Drahtsegementen des Drahtes (10) bildet und in Umfangsrichtung mindestens 6 Zellen, insbesondere mindestens 12 Zellen, insbesondere mindestens 16 Zellen, insbesondere mindestens 24 Zellen, insbesondere mindestens 36 Zellen, insbesondere mindestens 48 Zellen angeordnet sind, wobei für jede der vorstehend genannten Untergrenzen der Winkel (α) zwischen den Drahtsegmenten oder Drähten (10) und einer in Längsrichtung der Gitterstruktur verlaufenden Längsachse zumindest abschnittsweise jeweils mindestens 45°, insbesondere mindestens 50°, insbesondere mindestens 55°, insbesondere mindestens 60°, insbesondere mindestens 65°, insbesondere mindestens 70°, insbesondere mindestens 75° beträgt.

2. Funktionselement nach Anspruch 1,
**dadurch gekennzeichnet, dass**
die Gitterstruktur (12) im expandierten Ruhezustand einen Außendurchmesser von höchstens 7mm hat.

3. Funktionselement nach einem der Ansprüche 1 oder 2
**dadurch gekennzeichnet, dass**
der Draht (10) oder die Drähte jeweils einen Durchmesser von höchstens 120µm, insbesondere höchstens 90 µm, insbesondere höchstens 76 µm, insbesondere höchstens 51 µm, insbesondere höchstens 40 µm, insbesondere höchstens 35 µm, insbesondere höchstens 25 µm aufweisen.

4. Funktionselement nach einem der Ansprüche 1 bis 3,
**dadurch gekennzeichnet, dass**
eine Schiebekraft der Gitterstruktur (12) in einem Katheter höchstens 1,5N, vorzugsweise höchstens 1,4N, vorzugsweise höchstens 1,3N, vorzugsweise höchstens 1,2N, vorzugsweise höchstens 1,1N, vorzugsweise höchstens 1.0 N, vorzugsweise höchstens 0,9N, vorzugsweise höchstens 0,8N, weiter vorzugsweise, 0,7N, weiter vorzugsweise 0,6N, weiter vorzugsweise 0,5N jeweils pro 10 mm Länge in expandiertem Zustand beträgt, bei einem Katheter-Innendurchmesser von höchstens 0,8 mm, vorzugsweise höchstens 0,7 mm, vorzugsweise höchstens 0,6 mm, vorzugsweise höchstens 0,5 mm, vorzugsweise höchstens 0,4 mm.

5. Funktionselement nach einem der Ansprüche 1 bis 4,
**dadurch gekennzeichnet, dass**
die Mischoxidschicht im Bereich von Drahtüberkreuzung und/oder auf den Kontaktflächen der sich berührenden Drähte (10) der Gitterstruktur (12) ausgebildet ist.

6. Funktionselement nach einem der Ansprüche 1 bis 5,
**dadurch gekennzeichnet, dass**
die Schichtdicke mindestens 30nm, insbesondere mindestens 35nm, insbesondere mindestens 40nm, insbesondere mindestens 45nm, insbesondere mindestens 50nm, insbesondere mindestens 55nm beträgt.

7. Funktionselement nach einem der Ansprüche 1 bis 6,
**dadurch gekennzeichnet, dass**
die Schichtdicke höchstens 95nm, insbesondere höchstens 85nm, insbesondere höchstens 80nm, insbesondere höchstens 75nm, insbesondere höchstens 70nm, insbesondere höchstens 65nm, insbesondere höchstens 60nm beträgt.

8. Funktionselement nach einem der Ansprüche 1 bis 7,
**dadurch gekennzeichnet, dass**
der Peak der Sauerstoffkonzentration in der Mischoxidschicht ein Plateau bildet.

9. Funktionselement nach Anspruch 8,
**dadurch gekennzeichnet, dass**
das Verhältnis der Intensitäten zwischen Stickstoff und Sauerstoff N/O so eingestellt wird, dass das Verhältnis im Bereich des Sauerstoffplateaus höchstens 1:2,0, insbesondere aber von 1:2,5 bis 1:10 beträgt und zum Drahtmetallkörper hin abnimmt, wobei die Intensität jeweils durch Augerelektronenspektroskopie (AES) ermittelt wird.

10. Funktionselement nach einem der Ansprüche 1 bis 9,
**dadurch gekennzeichnet, dass**
Stickstoff ausgehend von der Schichtoberfläche bis in eine Tiefe der Mischoxidschicht von bis zu 2/6 der Gesamtdicke der Mischoxidschicht, insbesondere bis zu 3/6, insbesondere bis zu 4/6 der Gesamtdicke der Mischoxidschicht vorliegt.

11. Funktionselement nach einem der Ansprüche 1 bis 10,
**dadurch gekennzeichnet, dass**
ein Ni-Anteil der Mischoxidschicht in einem Bereich ausgehend von der Schichtoberfläche bis in eine Schichttiefe von 30% der Gesamtdicke der Mischoxidschicht höchstens 6 Gew.-% beträgt.

12. Funktionselement nach einem der Ansprüche 1 bis 11,
**dadurch gekennzeichnet, dass**
im inneren Bereich der Mischoxidschicht, der an den Drahtkörper angrenzt, eine Anreicherung von Nickeloxid ausgebildet ist.

13. System umfassend ein Funktionselement nach einem der Ansprüche 1 bis 12 sowie ein schlauchförmiges Element, insbesondere eine Schleuse, in der das Funktionselement angeordnet ist, wobei ein Innendurchmesser des schlauchförmigen Elementes insbesondere höchstens 0,8 mm, weiter insbesondere höchstens 0,7 mm, weiter insbesondere höchstens 0,6 mm, weiter insbesondere höchstens 0,5 mm, weiter insbesondere höchstens 0,4 mm ist.

## Claims

1. An intravascular function element having a tubular lattice structure (12) comprising a wire (10) or a plurality of wires (10), each of which consist or an alloy having the alloying elements nickel and titanium, wherein a mixed oxide layer containing TiO2 and at least one nitride, particularly titanium oxynitride or titanium nitride, with a layer thickness from 15 nm to 100 nm is formed on the surface of the wire (10) or wires (10), wherein the lattice structure forms cells from intersecting wires (10) or segments of the wires (10) in both the longitudinal and the circumferential direction thereof, and at least 6 cells, in particular at least 12 cells, more particularly at least 16 cells, more particularly at least 24 cells, more particularly at least 36 cells, more particularly at least 48 cells, are arranged in the circumferential direction, wherein for each of the aforementioned lower limits the angle (α) between the wire segments or wires (10) and a longitudinal axis extending in the lengthwise direction of the lattice structure is at least 45°, more particularly at least 50°, more particularly at least 55°, more particularly at least 60°, more particularly at least 65°, more particularly at least 70°, more particularly at least 75° respectively at least for sections thereof.

2. Functional element according to claim 1,
**characterized in that**
in the expanded state of rest the lattice structure (12) has an outer diameter of not more than 7 mm.

3. Functional element according to either of claims 1 or 2,
**characterized in that**
the wire (10) or wires each have a diameter of not more than 120 µm, particularly not more than 90 µm, more particularly not more than 76 µm, more particularly not more than 51 µm, more particularly not more than 40 µm, more particularly not more than 35 µm, more particularly not more than 25 µm.

4. Functional element according to any one of claims 1 to 3,
**characterized in that**
a sliding force of the lattice structure (12) in a catheter is not more than 1.5 N, preferably not more than 1.4 N, more preferably not more than 1.3 N, more preferably not more than 1.2 N, more preferably not more than 1.1 N, more preferably not more than 1.0 N, more preferably not more than 0.9 N, more preferably not more than 0.8 N, more preferably not more than 0.7 N, more preferably not more than 0.6 N, more preferably not more than 0.5 N for each 10 mm of length in the expanded state with an inner diameter of the catheter not exceeding 0.8 mm, preferably not exceeding 0.7 mm, more preferably not exceeding 0.6 mm, more preferably not exceeding 0.5 mm, more preferably not exceeding 0.4 mm.

5. Functional element according to any one of claims 1 to 4,
**characterized in that**
the mixed oxide layer is formed in the area of the wire intersection and/or on the contact surfaces of the touching wires (10) of the lattice structure (12).

6. Functional element according to any one of claims 1 to 5,
**characterized in that**
the layer thickness is at least 30 nm, particularly at least 35 nm, more particularly at least 40 nm, more particularly at least 45 nm, more particularly at least 50 nm, more particularly at least 55 nm.

7. Functional element according to any one of claims 1 to 6,
**characterized in that**
the layer thickness is not more than 95 nm, particularly a not more than 85 nm, more particularly not more than 80 nm, more particularly not more than 75 nm, more particularly not more than 70 nm, more particularly not more than 65 nm, more particularly not more than 60 nm.

8. Functional element according to any one of claims 1 to 7,
**characterized in that**
the peak of the oxygen concentration in the mixed oxide layer forms a plateau.

9. Functional element according to claim 8,
**characterized in that**
the ratio of intensities between nitrogen and oxygen N/O is adjusted so that the ratio close to the oxygen plateau is not more than 1:2.0, but particularly from 1:2.5 to 1:10 and decreases towards to the wire metal body, wherein the intensity is determined in each case by Auger electron spectroscopy (AES).

10. Functional element according to any one of claims 1 to 9,
**characterized in that**
starting from the layer surface to a depth of the mixed oxide layer nitrogen is present in a quantity of up to 2/6 of the total thickness of the mixed oxide layer, particularly up to 3/6, more particularly up to 4/6 of the total thickness of the mixed oxide layer.

11. Functional element according to any one of claims 1 to 10,
**characterized in that**
a Ni fraction of the mixed oxide layer does not exceed 6 % by weight in an area starting from the layer surface down to a layer depth of 30% of the total thickness of the mixed oxide layer.

12. Functional element according to any one of claims 1 to 11,
**characterized in that**
an enrichment of nickel oxide is formed in the inner area of the mixed oxide layer which is adjacent to the wire body.

13. System comprising a functional element according to any one of claims 1 to 12 as well as a tubular element, in particular a lock, in which the functional element is arranged, wherein an inner diameter of the tubular element is at most 0.8 mm, particularly at most 0.7 mm, more particularly at most 0.6 mm, more particularly at most 0.5 mm, more particularly at most 0.4 mm.

## Revendications

1. Élément fonctionnel intravasculaire comportant une structure grillagée en forme de tubule (12) qui présente un fil (10) ou plusieurs fils (10) respectivement composés d'un alliage contenant des éléments d'alliage nickel et titane, dans lequel une couche d'oxyde mélangée est réalisée sur la surface du fil (10)/des fils (10) en une épaisseur de couche de 15 nm à 100 nm qui contient du Ti02 et au moins un nitrure, en particulier du nitrure d'oxyde de titane et/ou du nitrure de titane, la structure grillagée formant respectivement, dans le sens longitudinal et dans le sens circonférentiel, des cellules constituées de fils se croisant (10) ou des segments de fil du fil (10) et étant disposées dans le sens circonférentiel au moins 6 cellules, en particulier au moins 12 cellules, en particulier au moins 16 cellules, en particulier au moins 24 cellules, en particulier au moins 36 cellules, en particulier au moins 48 cellules, sachant que, pour chacune des limites inférieures précitées, l'angle (α) entre les segments de fil ou les fils (10) et un axe longitudinal s'étendant dans le sens longitudinal de la structure grillagée, du moins par sections, 45°, est de respectivement au moins 50°, en particulier au moins 55°, en particulier au moins 60°, en particulier au moins 65°, en particulier au moins 70°, en particulier au moins 75°.

2. Élément fonctionnel selon la revendication 1, **caractérisé en ce que**
la structure grillagée (12), à l'état de repos expansé, a un diamètre extérieur de 7 mm au maximum.

3. Élément fonctionnel selon une des revendications 1 ou 2,
**caractérisé en ce que**
le fil (10) ou les fils présentent respectivement un diamètre de 120 µm au maximum, en particulier 90 µm au maximum, en particulier 76 µm au maximum, en particulier 51 µm au maximum, en particulier 40 µm au maximum, en particulier 35 µm au maximum, en particulier 25 µm au maximum.

4. Élément fonctionnel selon une des revendications 1 à 3,
**caractérisé en ce**
**qu'**une force de glissement de la structure grillagée (12) dans un cathéter s'élève respectivement à 1,5 N au maximum, de préférence 1,4 N au maximum, de préférence 1,3 N au maximum, de préférence 1,2 N au maximum, de préférence 1,1 N au maximum, de préférence 1,0 N au maximum, de préférence 0,9 N au maximum, de préférence 0,8 N au maximum, plus préférentiellement 0,7 N au maximum, plus préférentiellement 0,6 N au maximum, plus préférentiellement 0,5 N par 10 mm de longueur à l'état expansé, avec un diamètre intérieur du cathéter de 0,8 mm au maximum, de préférence 0,7 mm au maximum, de préférence 0,6 mm au maximum, de préférence 0,5 mm au maximum, de préférence 0,4 mm au maximum.

5. Élément fonctionnel selon une des revendications 1 à 4,
**caractérisé en ce que**
la couche d'oxyde mélangée est réalisée au niveau du croisement des fils et/ou sur les surfaces de contact des fils se touchant (10) de la structure grillagée (12).

6. Élément fonctionnel selon une des revendications 1 à 5,
**caractérisé en ce que**
l'épaisseur de la couche s'élève à au moins 30 nm, en particulier au moins 35 nm, en particulier au moins 40 nm, en particulier au moins 45 nm, en particulier au moins 50 nm, en particulier au moins 55 nm.

7. Élément fonctionnel selon une des revendications 1 à 6,
**caractérisé en ce que**
l'épaisseur de la couche s'élève à 95 nm au maximum, en particulier à 85 nm au maximum, en particulier à 80 nm au maximum, en particulier à 75 nm au maximum, en particulier à 70 nm au maximum, en particulier à 65 nm au maximum, en particulier à 60 nm au maximum.

8. Élément fonctionnel selon une des revendications 1 à 7,
**caractérisé en ce que**
la crête de la concentration d'oxygène dans la couche d'oxyde mélangée forme un plateau.

9. Élément fonctionnel selon la revendication 8, **caractérisé en ce que**
le rapport des intensités entre l'azote et l'oxygène N/O est paramétré de manière à ce que ce rapport soit au niveau du plateau d'oxygène de 1 à 2,0 au maximum, mais en particulier de 1 à 2,5 jusqu'à 1 à 10 et diminue en direction du corps en fil métallique, l'intensité étant respectivement déterminée par spectroscopie électronique Auger (SEA).

10. Élément fonctionnel selon une des revendications 1 à 9,
**caractérisé en ce que**
de l'azote émanant de la surface de la couche est présent jusqu'à une profondeur de la couche d'oxyde mélangée représentant jusqu'à 2/6 de l'épaisseur totale de la couche d'oxyde mélangée, en particulier jusqu'à 3/6, en particulier jusqu'à 4/6 de l'épaisseur totale de la couche d'oxyde mélangée.

11. Élément fonctionnel selon une des revendications 1 à 10,
**caractérisé en ce**
**qu'**une proportion de Ni de la couche d'oxyde mélangée dans une zone partant de la surface de la couche jusqu'à une profondeur de couche représentant 30 % de l'épaisseur totale de la couche d'oxyde mélangée représente 6 % en poids au maximum.

12. Élément fonctionnel selon une des revendications 1 à 11,
**caractérisé en ce que**,
dans la partie intérieure de la couche d'oxyde mélangée qui est adjacente au corps en fil, un enrichissement par oxyde de nickel est réalisé.

13. Système comprenant un élément fonctionnel selon une des revendications 1 à 12 et un élément de forme tubulaire, en particulier un sas, dans lequel l'élément fonctionnel est disposé, un diamètre intérieur de l'élément de forme tubulaire étant en particulier de 0,8 mm au maximum, plus notamment de 0,7 mm au maximum, plus notamment de 0,6 mm au maximum, plus notamment de 0,5 mm au maximum, plus notamment de 0,4 mm au maximum.
